# EUROPEAN PATENT SPECIFICATION

(11) **EP 0 346 998 B1**
(45) Date of publication and mention of the grant of the patent: **22.09.1993**
(21) Application number: 89201537.1
(22) Date of filing: 14.06.1989
(51) Int. Cl.: G01N 33/569, G01N 33/577

(54) **Method of demonstrating Marek virus in poultry**
Verfahren zum Nachweis des Marek-Virus in Geflügel
Procédé pour la détection du virus de Marek chez la volaille

(30) Priority: 16.06.1988 NL 8801534
(43) Date of publication of application: 20.12.1989
(73) Proprietor: DUPHAR INTERNATIONAL RESEARCH B.V, NL-1381 CP Weesp (NL)
(72) Inventor: Scholten, Rudolf, Weesp (NL); Hilgers, Lucas A.T., Weesp (NL); Weststrate, Marinus W., Weesp (NL)
(74) Representative: Muis, Maarten

(56) References cited:
- BIOLOGICAL ABSTRACTS, vol. 79, no. 9, 1985, Philadelphia, PA (US); Y.-Q. CHENG et al., p. 425, no. 77194#
- CHEMICAL ABSTRACTS, vol. 101, no. 11, 10 September 1984, Columbus, OH (US); K.R. WEHMEYER, p. 291, no. 86557a#
- CHEMICAL ABSTRACTS, vol. 100, no. 7, 13 February 1984, Columbus, OH (US); I. KAZUYOSHI et al., p. 301, no. 48184y#
- CHEMICAL ABSTRACTS, vol. 101, no. 15, 08 October 1984, Columbus, OH (US); R.F. SILVA et al., p. 367, no. 126480d#
- CHEMICAL ABSTRACTS, vol. 101(II), 1984, Columbus, OH (US); p. 291, no. 86547x#
- VETERINARY VIROLOGY, chapter 19, Academic Press Inc., 1987; F. FENNER et al., pp. 366-373#

## Description

The invention relates to a method of demonstrating Marek virus in poultry according to the ELISA principle and to a kit with which this method can be carried out.

Poultry which become infected with Marek Disease virus (MDV) start secreting virus 2-4 weeks after the infection. Said secretion of the virus takes place via the skin and the feather follicles. By secretion of cell-free virus, other animals are infected (horizontal spreading).

Up till now, MDV is demonstrated by means of the agar gel precipitation (AGP)-test (Avian Pathol. 4, (1975), 147-162), or by means of the enzyme linked immunosorbent assay (ELISA), (J. Virol.Meth. 14, (1986), 237-241 and 13, (1986), 231-244 and Biological Abstracts, (1985), p.425, 79 (9) : 77194). These known tests suffer from the disadvantage that they take quite some time (24-48 hours), that special apparatus is required, and that they can be carried out only in the laboratory. Further state of the art relating to MDV is also disclosed in Veterinary Virology, by Frank Fenner el al., Academic Press Inc., (1987) chapter 19 pp. 366-373, as well as in Chemical Abstracts, (1984), p. 301, 100 (7), abstract N^{o} 48184 y

It has been found that the determination of MDV according to the ELISA principle can also he carried out by using as a carrier a feather of the animal to be examined.

The invention therefore relates to a method of demonstrating MDV in poultry according to the ELISA principle in which a feather is used as a carrier by fixing the follicular debris with a fixative on the feather tip, incubating the feather with an antibody directed against the MDV itself or against an antigen induced by MDV, and an enzyme coupled thereto; and finally incubating the feather with a substrate suitable for the enzyme. The presence of MDV appears from a macroscopically observable discoloring on the feather tip which arises from reaction of the enzyme with the substrate.

According to a preferred embodiment the feather, after having been treated with a suitable fixative, is first incubated with a monoclonal antibody directed against MDV, is then incubated with a second antibody against the said antibody and to which an enzyme is coupled, and finally with a substrate suitable for the enzyme. The advantage of the said method is that the method is extremely selective.

A suitable fixative is, for example, a solution in 70% ethanol which comprises 0.3% of hydrogen peroxide and 1% of sodium azide.

As a monoclonal antibody (mcAb) directed against MDV may be used, for example, mcAb 35.1. mcAb 35.1 can be obtained by immunizing mice intraperitoneally with 10⁸ ethanol-fixed MDCC-MSB₁ cells in PBS (0.01 M; pH 7.4), and boostering after 3 weeks with the same amount of antigen. Two weeks later the mice are boostered intravenously with 10⁶ MDCC-MSB₁ cells, and three days after this booster spleen cells are collected and fused with Sp2/0-Ag14 myeloma cells, according to the method of Köhler et al, Nature 256, (1975), 495-497, using PEG 4000 as fusogene. Immunofluorescence staining on MDV-infected chicken embryo fibroblasts (MDV-CEF) can be used to screen developing clones for anti-MDV activity. Clone 35.1 is subcloned twice by limiting dilution.

Mcab 35.1 is directed against a none-structural cellular protein induced by MDV. This mcAb 35.1 can be demonstrated by means of goat-antimouse-peroxidase in 10% normal goat serum in phosphate buffered physiological saline solution (PBS). As a substrate suitable for peroxidase may be used, for example, a solution of 0.5 mg/ml of diaminobenzidine in PBS which comprises 0.006% of hydrogen peroxide.

The invention also relates to a kit suitable for carrying out the method according to the invention.

As with the two known methods the 3 serotypes in which MDV occurs can be demonstrated by means of the method according to the invention. Vaccine virus (CVI 988) which also spreads horizontally is, however, not detected in the method. As a result of this it is possible to demonstrate field virus in the follicular debris on the feather tip by means of the said method.

Since taking samples, carrying out and reading the test according to the invention is very simple and can also be carried out outside the laboratory in a short period of time (approximately 2 hours), the said method is suitable for use in a diagnostic kit in the field which can be used by the veterinarian or the stockbreeder.

The invention will now be described in greater detail with reference to the ensuing specific example.

### EXAMPLE

Feathers of animals to be examined were treated, immediately after the removal, with a solution of 0.3% of hydrogen peroxide and 1% of sodium azide in 70% of ethanol for 30 minutes so as to fix the follicular debris on the feather tip. The feathers were then treated for 10 minutes with 1 ml of 10% of normal goat serum in PBS and incubated for 30 minutes with 200 µl of supernatant of mcAb 35.1. Incubation was always carried out at room temperature in 1 ml-tubes of polypropylene. Between the incubation steps the feathers were washed three times with PBS.

Adsorbed mcAb was demonstrated by incubating the feathers for 30 minutes in 200 µl of goat-antimouse-Ig-peroxidase (1/50 diluted in 10% of normal goat serum in PBS). After washing with PBS there was incubated for 15 minutes with 0.5 mg/ml of diaminobenzidine and 0.006% of hydrogen peroxide.

The feathers originating from MDV-infected animals show clearly observable brown spots on the feather tip. Feathers of non-infected animals do not show these spots.

## Claims

1. A method of demonstrating Marek Disease Virus (MDV) in poultry, characterized in that the determination is carried out by using as a carrier a feather of the animal to be examined, fixing the follicular debris with a fixative on the feather tip, reacting the feather with an antibody directed against MDV itself or against an antigen induced by MDV, and demonstrating the bound antibody.

2. A method as claimed in Claim 1, characterized in that the feather is incubated with an antibody directed against the MDV and an enzyme coupled thereto and the feather is then incubated with a substrate suitable for the enzyme.

3. A method as claimed in claim 1, characterized in that the feather is incubated with a monoclonal antibody directed against the MDV, is then incubated with a second antibody directed against the first antibody and to which the enzyme is coupled, and is finally incubated with a substrate suitable for the enzyme.

4. A method as claimed in Claim 3, characterized in that the goat-anti-mouse-peroxidase is used as a second antibody.

5. A method as claimed in Claim 3, characterized in that the substrate used is PBS with 0.5 mg/ml of diaminobenzidine and 0.006% of hydrogen peroxide.

6. A kit suitable for carrying out the method as claimed in Claim 1, comprising inter alia a fixative, a blocking protein, a monoclonal antibody directed against MDV and to which an enzyme is coupled, a substrate for the enzyme and optionally directives with prescription to carry out the determination.

7. A kit as claimed in claim 6, comprising inter alia a fixative, a blocking protein, a monoclonal antibody directed against MDV, and antibody directed against the said monoclonal antibody with an enzyme coupled thereto, a substrate for the enzyme and optionally directives for carrying out the determination.

## Patentansprüche

1. Ein Verfahren zum Nachweis des Marek-Disease-Virus (MDV) bei Geflügel, dadurch gekennzeichnet, daß die Bestimmung dadurch durchgeführt wird, daß man eine Feder des zu untersuchenden Tieres als einen Träger verwendet, die follikulären Abriebteilchen mit einem Fixierungsmittel auf der Federspitze fixiert, die Feder mit einem Antikörper, der gegen das MDV selbst oder ein Antigen, das durch MDV induziert wurde, gerichtet ist, umsetzt; und den gebundenen Antikörper nachweist.

2. Ein Verfahren wie in Anspruch 1 beansprucht, dadurch gekennzeichnet, daß die Feder mit einem gegen das MDV gerichteten Antikörper und einem daran gekoppelten Enzym inkubiert wird und die Feder dann mit einem für das Enzym geeigneten Substrat inkubiert wird.

3. Ein Verfahren wie in Anspruch 1 beansprucht, dadurch gekennzeichnet, daß die Feder mit einem gegen das MDV gerichteten monoklonalen Antikörper inkubiert wird, dann mit einem zweiten gegen den ersten Antikörper gerichteten Antikörper, an den das Enzym gebunden ist, inkubiert wird und schließlich mit einem für das Enzym geeigneten Substrat inkubiert wird.

4. Ein Verfahren wie in Anspruch 3 beansprucht, dadurch gekennzeichnet, daß die Ziegen-Antimaus-Peroxidase als zweiter Antikörper verwendet wird.

5. Ein Verfahren wie in Anspruch 3 beansprucht, dadurch gekennzeichnet, daß das verwendete Substrat PBS mit 0.5 mg/ml Diaminobenzidin und 0.006% Wasserstoffperoxid ist.

6. Ein zur Durchführung des in Anspruch 1 beanspruchten Verfahrens geeignetes Kit, das unter anderem ein Fixierungsmittel, ein Blockierungsprotein, einen gegen MDV gerichteten monoklonalen Antikörper, an den ein Enzym gekoppelt ist, ein Substrat für das Enzym und gegebenenfalls Anweisungen mit einer Vorschrift zur Durchführung der Bestimmung umfasst.

7. Ein Kit wie in Anspruch 6 beansprucht, das unter anderem ein Fixierungsmittel, ein Blockierungsprotein, einen gegen MDV gerichteten monoklonalen Antikörper, und einen Antikörper gerichtet gegen den monoklonalen Antikörper mit einem daran gekoppelten Enzym, ein Substrat für das Enzym und gegebenenfalls Anweisungen mit einer Vorschrift zur Durchführung der Bestimmung umfasst.

## Revendications

1. Procédé pour mettre en évidence le virus de la maladie de Marek (MDV) chez la volaille, caractérisé en ce qu'on procède à cette mise en évidence en utilisant comme support une plume de l'animal à examiner, en fixant les débris folliculaires, à l'aide d'un fixatif, sur l'extrémité des plumes, en faisant réagir la plume avec un anticorps dirigé contre le MDV proprement dit ou contre un antigène induit par le MDV, et en mettant en évidence l'anticorps lié.

2. Procédé selon la revendication 1, caractérisé en ce que la plume est incubée avec un anticorps dirigé contre le MDV et une enzyme qui y est couplée, et la plume est ensuite incubée avec un substrat convenant à l'enzyme.

3. Procédé selon la revendication 1, caractérisé en ce que la plume est incubée avec un anticorps monoclonal dirigé contre le MDV, puis est incubée avec un deuxième anticorps dirigé contre le premier anticorps et auquel est couplée l'enzyme, et est finalement incubée avec un substrat convenant à l'enzyme.

4. Procédé selon la revendication 3, caractérisé en ce qu'on utilise comme deuxième anticorps de la peroxydase anti-souris de chèvre.

5. Procédé selon la revendication 3, caractérisé en ce que le substrat utilisé est le PBS avec 0,5 mg/ml de diaminobenzidine et 0,006 % de peroxyde d'hydrogène.

6. Coffret convenant à la mise en oeuvre du procédé selon la revendication 1, comprenant entre autres un fixatif, une protéine de blocage, un anticorps monoclonal dirigé contre le MDV et auquel est couplée une enzyme, un substrat pour l'enzyme et éventuellement des instructions de mise en oeuvre de l'opération.

7. Coffret selon la revendication 6, comprenant entre autres un fixatif, une protéine de blocage, un anticorps monoclonal dirigé contre le MDV et un anticorps dirigé contre ledit anticorps monoclonal et avec une enzyme qui y est couplée, un substrat pour l'enzyme et éventuellement des instructions pour la mise en oeuvre de l'opération.
